Europäisches Patentamt

European Patent Office

Office européen des brevets

(19)

(11) Publication number: **0 230 155**
**A1**

# EUROPEAN PATENT APPLICATION

(21) Application number: 86401894.0

(22) Date of filing: 28.08.86

(51) Int. Cl.⁴: **A61B 6/06** , **A61B 6/03** ,
**G01T 1/164**

(30) Priority: 26.11.85 JP 266588/85

(43) Date of publication of application:
29.07.87 Bulletin 87/31

(84) Designated Contracting States:
DE GB

(71) Applicant: **SHIMADZU CORPORATION**
**378, Ichinofunairi-cho Kawaramachi-dori Nijo**
**Sagaru**
**Nakagyo-ku Kyoto 604(JP)**

(72) Inventor: **Kiri, Motosada**
**14-11, 6-Chome Ooe Nishi Shinbayashi-cho**
**Nishikyo-ku Kyoto(JP)**
Inventor: **Nakanishi, Takeshi**
**1-53, Uzumasa Sujaku-cho**
**Ukyo-ku Kyoto(JP)**
Inventor: **Shibata, Kenji**
**311 Mibu Grando Haitsu 4 Chudoji**
**Kagita-cho**
**Shimogyo-ku Kyoto(JP)**

(74) Representative: **Rodhain, Claude et al**
**Cabinet Claude Rodhain 30, rue la Boétie**
**F-75008 Paris(FR)**

(54) Apparatus for radiography.

(57) The invention concerns an apparatus for diagnostic radiography comprising a radiant source (I) - (an X-ray tube), a slit (9) for controlling the X-ray beam on one side of an object body (6) and a plurality of X-ray sensor rows on the other side of the object body (6). X-rays transmitted through the body are received by the sensors and are processed on a digital basis to define an image picture of the body. The sensors are lined in a plurality of rows and are subject to continuous exposure by the X-ray beam during the scanning time for completing one image pattern. The digital image data for the body is formed during the scanning time by accumulation of signals from the X-ray sensors which define respectively the image elements. The accumulation is built up by addition of signals from X-ray sensors which come in agreement of sensing line with the scanning spot. This invention brings about improvement in sensitivity, shorter scanning time, and enhancement of resolution due to smaller intervals between the scanning lines.

FIG 4

## Apparatus for radiography

### Field of the Invention

This invention relates to an apparatus for diagnostic radiography. In particular, this relates to an apparatus for direct formation of a digital radiographic image with the aid of a scanning radiant sensor array.

### Background of the Invention

Diagnosis by X-ray image has a long history of development with application of the art involved in photography and has reached high-level technology as well as wide prevalence thanks to progress in X-ray image intensifier, television-related systems and also to the advent of digital imaging with use of image accumulation plate. And it is noteworthy that, in process of obtaining a digital image, these recent art heretofore involve steps of obtaining first an analog image which will then be converted into digital. On the other hand, apparatus for digital-imaging with slit X-ray method have been known as featuring direct formation of digital X-ray image, which comprise providing a radiant source - (X-ray source) cooperating with an object body placed between a slit system at one side and a radiant sensor array at the other side for penetrative photographing and wherein the X-ray beam irradiating in form of planar sector by control of the slit is allowed to be propagated through the object body and is then received by the linearly arrayed sensors while concurrently X-ray beam and sensors are moved with a certain speed.

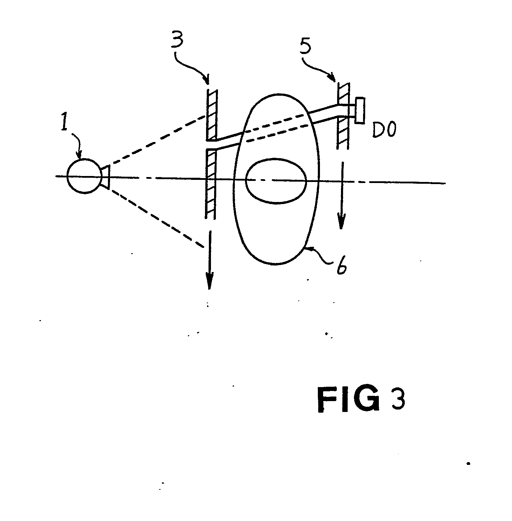

Fig. 3 is a schematic diagram for explaining principles of conventional apparatus for digital-imaging with slit X-ray wherein an X-ray beam irradiated from a X-ray tube I is controlled to form a beam having a lateral slot form in section by passing through a slit 3 disposed in front of an object body 6 through which the beam is then propagated. The width of the slit 3 is preferentially made smaller than that of a slit provided on a shielder 5 which is disposed behind the object 6. This shielder may be chosen in relation with the degree of recuction of scattered ray and the utilization factor of X-ray.

The radiant sensors DO consist, for instance, of a scintillator or several photodiodes having accumulative capacity of image or any other semiconductor elements, and being arrayed perpendicular to the drawing so as to cover the scope of view desired. During the completion of each image pattern, X-ray is continuously radiated and the slit 3, the shielder 5, and the radiant sensors DO are moved, with mutual positioning relationship being maintained perpendicularly to the sensor array with a certain speed and the respective sensor elements are designed to alternate readout and accumulation of image data.

However, the above photographing method has difficulty in attaining higher resolution and higher processing speed. More precisely, the smaller the areas of respective sensor elements are made to attain higher resolution, the more compensation becomes necessary of decrease in sensibility due to the reduction of the X-ray exposed area which would require a longer scanning time Hence conflicting requirements or antinomy.

In connection thereto, prior art which allows enhancement in resolution without need of longer scanning time is disclosed in Japanese Unexamined Patent Publication 230540/1984, wherein a plurality of linear X-ray sensor rows are provided in cooperation with one slit having a width corresponding to said plural rows of sensors. These sensors and the slit are adapted to move together perpendicularly to the sensor rows with intermittent motions of making stops after one pitch move which ranges said plural rows and wherein at respective stops X-ray is irradiated in order to obtain aimed X-ray images.

However, this prior art needs intermittent moves which do not always allow shorter scanning time.

Furthermore, in the above X-ray digital slit radiography, signals transmitted from each sensor element are analog voltages or currents, which will be integrated during scanning times with respect to respective image elements and then converted to digital to serve as image data of respective image elements In contrast, a method is known of counting x-ray photons incident to sensors, which method is thought to be the most reliable in measuring intensity of any radiative light because X-ray and other radiations are intrinsically of particle nature.

As an example of digital radiography of the type of counting X-ray photons or light quantums, there is art disclosed in European Patent Application 84ll2l0l.5 (Publication n° 0l37487), for instance, wherein many radiant sensors and counting devices directly connected thereto are rowed in one array and thereby photographing area to be projected with transmitted X-ray will be scanned and, as scanning moves over respective counting channels, counts at respective sensors are as one unit transferred to corresponding memory addresses (cells), which will finally build up a two dimensional image.

But the drawbacks inherent to the above method lie in the littleness of exposure area with sensors which will imply poor sensitivity and longer radiographing (or exposure) time, hence failure in practical use. Specifically, in the case that a possible largest X-ray tube is applied to output 100 mR/sec and sensor elements of 0.35 square mm are used to exposure thereto, the number of photons to be caught by each sensor comes to be about 3675 particles per second. On the other hand, in the case of diagnosis of human bodies wherein transmission rate is assumed 10 % and for the reason that 100000 particles of sensed photons are necessary in order to restrict a noise level below 1 % based on statistical variations, it is concluded in the above case that necessary exposure time is about 27 seconds which is too long for patients to stop their respirations and also affection due to subtle move of human body is probable for such long seconds.

## Summary of the Invention

For the purpose of overcoming the drawbacks as noted above, this invention proposes an apparatus for radiographing an object body comprising a X-ray source, at least one slit on one side of said object body for controlling diffusion of X-ray emanated from the source, rows of sensors on the other side of the object body for detecting the transmitted X-ray, said apparatus further comprising drive means for moving the X-ray source, the slit and the sensor rows relatively to said object body while maintaining their relative position unchanged, so as to form an image on said rows of sensors by scanning said object body with said X-rays characterized in that said sensors are lined up in a plurality of rows and are submitted to continuous exposure to X-ray during the scanning time corresponding to completing one image pattern, digital image data for the image of said object body being formed during said scanning time by accumulation (integration or counting) of signals from the x-ray sensors defining image elements. Preferentially said accumulation is built up by addition of signals from X-ray sensors which come in agreement of sensing line therein with scanning spot.

Further, this invention proposes particular location of a pair of sensor rows wherein positions of sensors belonging to different rows are mutually staggered or shifted by half so that the scanning line along one sensor row and the same along the adjacent row of the same pair will be interlaced or interrelated, whereby sensitivity, scanning time and image resolution will be improved in application to radiography with use of scanning digital imaging method.

Here, it is to be noted that, in this invention, scanning is conducted in image-sensing devices to build up an image and therefore scanning of X-ray beam is not referred to hereinbelow, although scanning of x-ray beam is allowed as alternative therefor.

## Brief Explanation of the Drawings

Fig. 1 is a schematic diagram to show a first embodiment of the apparatus for radiography according to the invention.

Fig. 2 shows a time chart for scanning applied to the first embodiment.

Fig. 3 shows a schematic diagram useful to explain conventional X-ray radiography of the type of digital slit method.

Fig. 4 is a perspective view to show whole arrangements of a second embodiment of the inventive apparatus for radiography.

Fig. 5 is a schematic diagram to show devices disposed in periphery of sensors in the second embodiment.

Fig. 6 is a diagram useful for explaining positions of sensor rows in the second embodiment of Fig. 4.

Fig. 7 is a diagram useful for explaining scanning process in same embodiment.

## Description of the Inventive Embodiments

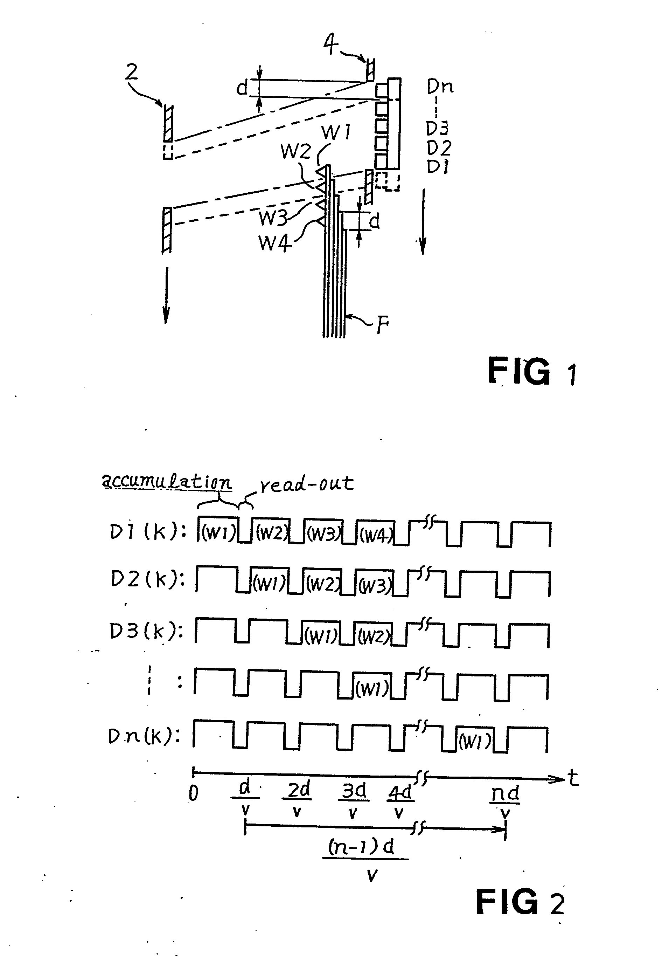

Fig. 1 shows a first embodiment of the present invention, wherein X-ray sensors are positioned in dispositionsas noted DI, D2, D3, ... Dn in parallel to the drawing and also in array disposition (not shown) perpendicularly to the same so as to form as a whole a matrix of sensors. Ladder phantom F, dummy for an object body, is drawn to explain relationship between sensors and X-ray image data wherein references to indicate stepping thicknesses, WI, W2, W3, ... Wn, are given from the thinnest step portion.

Fig. 2 is a chart to show behaviors of sensors in accumulation (integration) and read-out of image data which arise incidentally to scanning. The embodiment of Fig. 1 is adapted to move slit 2, shielder 4 and X-ray sensors in keeping their mutual positional relations unchanged and Fig. 2 shows behaviors when those moving members move with a speed v (direction is assumed to be downwards as indicated by arrow) wherein, as time course progresses as noted in horizontal axis, respective

sensors, DI, ... Dn, accumulate X-ray image data at high level timings and read out at low level timings with respect to WI, W2, W3, ... portions of the phantom F. And therein Dn(k) means a sensor positioned in row n, column k and further (WI), (W2), ... indicate image data at the step portions of WI, W2, ... and these image data will be subject to addition subsequent to read-out. Consequently, time requirement is (n -I)d/v for scanning one image element for an object body wherein d is sensor height which is equal to that of step length W as shown in Fig. I. And Fig. I shows a status where only WI portion is in the act and broken lines indicate the next status by downward move.

Addition of data from respective sensors may be performed by circuits connected therebehind which are similar to those to be explained in connection with a second example. And, if CCD is used as sensor and if transfer of signal charge is allowed to follow sequence of DI, D2, D3, ... Dn, the charge transfer speed and sensor move speed may be accorded to each other and thereby addition processing may be performed without external additional circuit which helps making simple the necessary circuits. Slit and drive means for moving the sensors are similar to those employed in the second example.

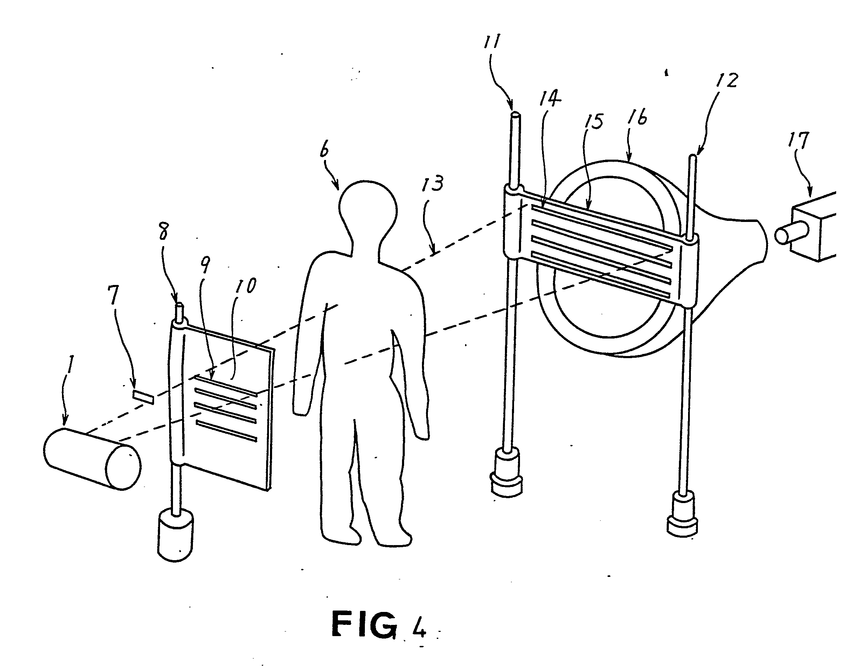

Next, reference is made to the second example of this invention, of which systematic arrangement is shown in perspective in Fig. 4 wherein I is a X-ray tube, 6 is an object body, 7 is a radiant dose monitor to monitor X-ray intensity, 8 is a post to support a slit plate on which a plurality of slits 9 are blanked and wherein X-ray emanated from the tube I passes through the slit 9 to form a diverging beam I3 which penetrates through the object body 6 and is sensed by respective sensors in array I4 on the sensor plate I5. I6 is a X-ray image intensifier (XII) for monitor use. I7 is a television camera to photograph an image on the intensifier (XII). The slit plate I0 and the sensor plate I5 are adapted to move up and down along the posts II, I2, 8. Drive means for such moves is not shown because such a mechanism is not important to the present invention, though, for example, it may be effected by providing screw thread cut on the posts 8 and II and also providing engageable screw thread cut on contact portion of the slit plate I0 and the sensor plate I5 and, motors being set at the base of the posts 8 and II to revolve them synchronously, thereby smooth vertical movements may be realized of the plates 10 and I5. Of course, X-ray beam I3 which has passed the slit 9 should always be regulated so as to be incident on sensor array I4, for which mutual speeds of both plates are to be controlled.

Movements involved in the above notes are meant for changing positions where X-ray beam penetrates the object body. Accordingly, only the relative positions and moves matter, which means for instance that the X-ray tube I may be moved in place of the slit plate I0.

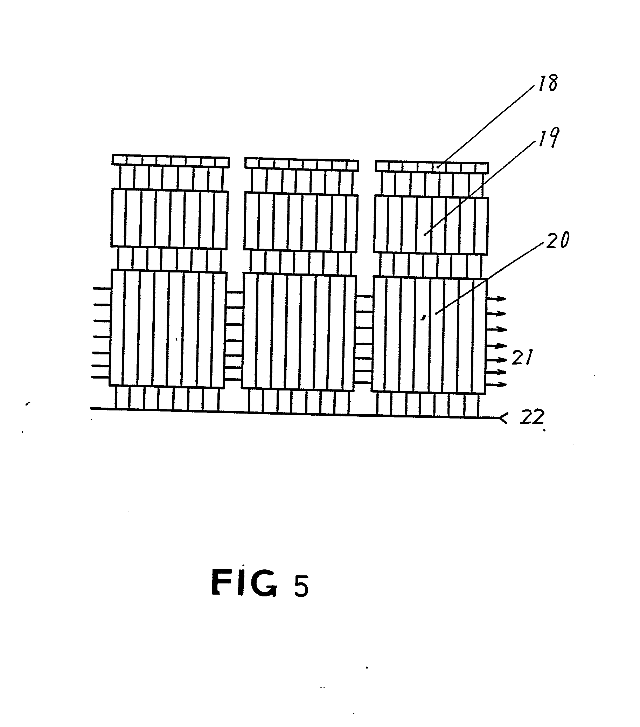

Fig. 5 is an enlarged diagram to show structures on the sensor plate I5 in Fig. 4 wherein I8 is a sensor (a detector element), I9 is an amplifier for amplifying signals from the sensor I8, 20 is a counter to count sensed signal pulses. The sensor I8, the amplifier I9 and the counter circuit 20 define one system flow and herein seven (7) systems define one block, and laterally a plurality of these blocks are lined up to form one row.

Signal lines 2I are data bus to input data from the counter 20 to subsequent image treatment areas and signal lines 22 are address bus to indicate the counter 20 to be read out. Each of these blocks comprising sensors, amplifiers and counters is formed on one piece of semiconductor chip. The sensor elements may be a semiconductor element, for instance, CdTe (cadmium, tellurium) or a combination of scintillator and photodiode, which would be decided in view of radiant energy and species.

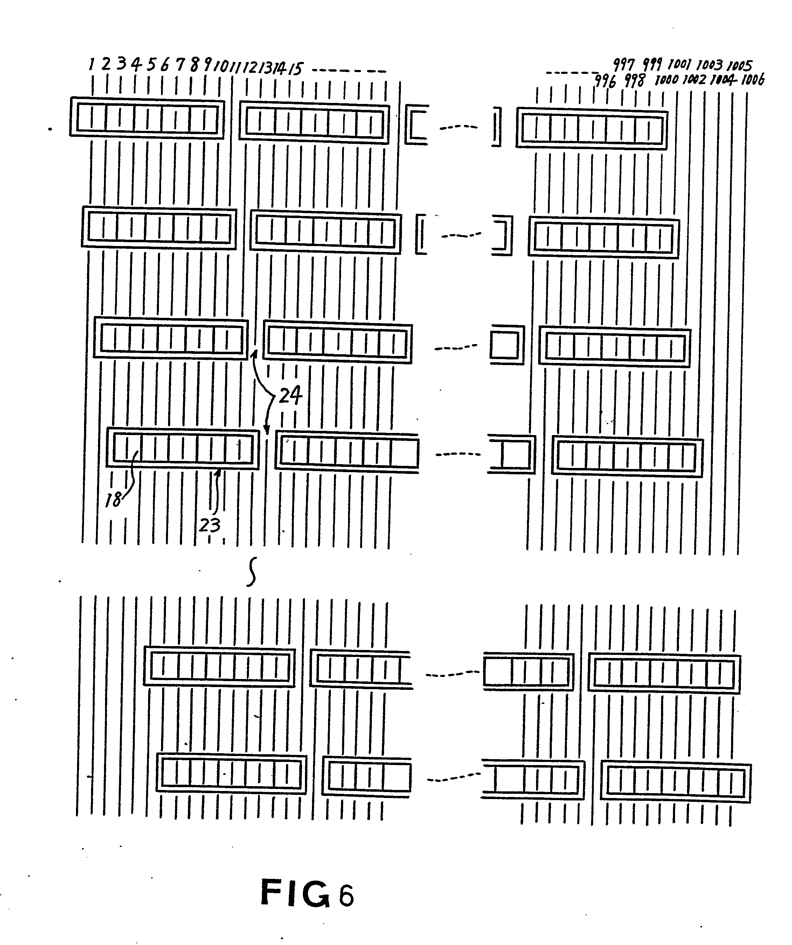

Fig. 6 shows arrangement of radiant sensors used in the second embodiment, wherein 500 pieces (including absent positions) of square sensors (detector element) are lined up to define one linear array or first row and, with an interval of about 10 mm inbetween , a second row is similarly lined up in parallel these two rows defining one pair of interlaced rows, corresponding to one slit on the slit plate, wherein one piece of actual sensor measures a square having a side = 0.7 mm (= 350 mm/500 pieces) and the two rows are positioned to be shifted longitudinally by one image unit, that is, 0.35 mm, half the side, so that odd numbered sensing lines extending vertically may run through centers of the first row squares (even numbered run through borders between two adjacent ones of the first row) and that even numbered sensing lines also vertically extending may run through centers of the second row squares (odd numbered run through borders between two adjacent ones of the second row) and thereby odd numbered sensing areas of the first row and even numbered sensing areas of the second row are connected to form a staggering sensing route or interlacing configuration. Fig. 6 shows an arrangement comprising four (4) pairs from first to fourth, each consisting of two rows in parallel with appropriate interval, wherein five sensors I8 in row are grouped to one block 23 and therefore one lateral scanning line ranges over vertical sensing lines numbered from I to I006. 24 is a void or absent portion which is due to the combination of two adjacent blocks 23 to form one row.

The X-ray exposure area of this arrangement is equal to four (4) times the exposure area of an arrangement comprising conventional simple sensors which are assumed being squares having 0.35 mm side, and further this is equal to four (4) times in relation to four (4) groups of rows, hence a 16 times rise in sensibility is assumed and for which times logically photographing time can be made shorter. Further, a X-ray tube can output X-ray several times stronger for short time operation than rated output for long time operation. Addition of this merit to the further above will make it possible to shorten necessary time for photographing down to 0.5 second, which time might lie within tolerable range for pause of human respirations and thereby sharp images might be obtained without affection of blood flow except for neighborhood of the heart. In conclusion, photographing time is reduced below 1/50 while the increase in the number of sensors is held within four times.

Although it is true that resolution at finer level reduces with increased area of one sensing element, in the present case, it is not as much as halved in view of the focal distance of the X-ray source. In place, positive point exceeds, by overlapping sensing areas mutually and therefore avoiding loss of information lying interface between adjacent elements and thereby more detailed display of an image at finer level is attained as merit. For instance, when some sharp periphery of an image appears oblique on an image screen and when conventional small-size sensors are used, conventional sensors would reproduce such peripheral lines in angular stepping lines as image, instead of smooth one. The present art, because adjacent sensing regions are somewhat overlapped, can receive such an oblique line over adjacent plural element areas so that a smoothly varying line may be reproduced, which will bring a viewer an easier pattern recognition of a finer image.

Furthermore, in manufacture of sensor devices, large size semiconductor material is not always available, and then normally several smaller array blocks are connected which cause data lacking areas at the connecting spots, for which a countermeasure is required. In addition, because of uneven or irregular characteristics with respective elements and related amplifiers, generally, sensibilities with respective elements become different to each other and thereby local unevenness will appear in the image obtained. This trouble can be amended by corrective calculation in reference to data obtained with even image which may be measured with use of evenly illuminating X-ray ex ante or ex post. In this invention wherein positioning is so devised as to fill gaps between a plurality of sensor arrays by shift by half or by prevention of positional agreement, unevenness is dispersed and corrective calculation for remaining unevenness will be more effective.

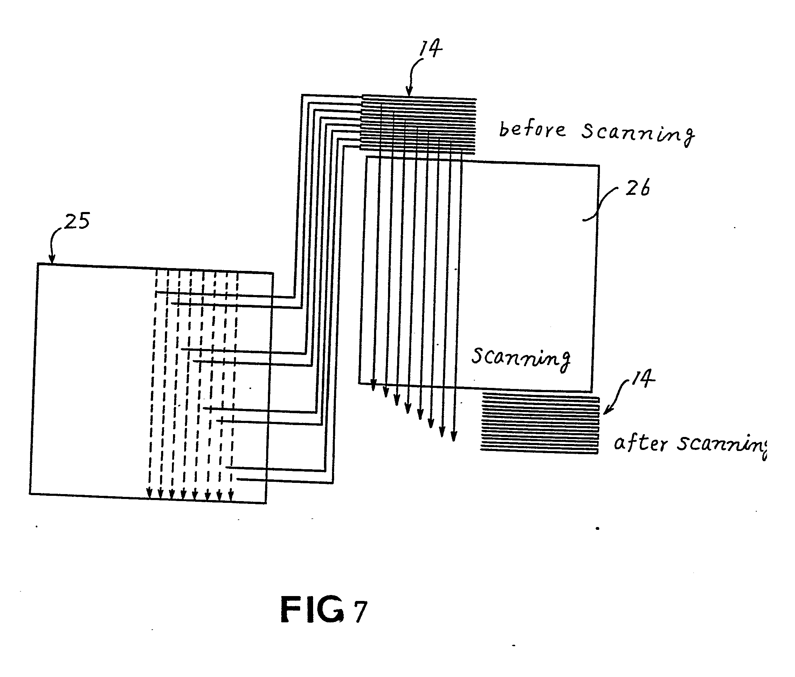

Fig. 7 shows processing circuits of data obtained by sensors 14 (see the like in Fig. 4), wherein, as a viewing scope 26 is scanned from upper to lower, respective sensor data (counts) are transferred to an image processing zone, specifically, to corresponding memory addresses to store, in an image memory 25. An incident aperture is so set that these sensors zone is only exposed to X-ray radiation which status should be maintained in movements during measurement. And sensors and connected counters are adapted to measure intensity or to count pulses of transmitted X-ray from an object body as movements progress and pulse counts are transferred to the image zone, specifically, to corresponding addresses wherein counts are accumulated and finally the whole image is built up. Interval between sensor rows are set to be large enough (more than one element) to avoid detrimental side X-ray scattering around the transmitted X-ray, but not to be too large to need too much time in scanning the same locations because of this interval;

Next refers to a problem which should be solved in practical use in connection with scanning type, that is, the correction of unevenness in shading with an image mainly due to timewise fluctuation with power output from a X-ray source. The main cause for the fluctuation with a X-ray source comes from the remaining pulsation in rectified currents. Normally, in case of three phase full-wave rectification, variation is contained within 25 %. This trouble does not appear in conventional nonscanning radiography which produces an image covering the whole view scope instantaneously and for that account, unevenness with image is not involved. But the present invention as any other employing scanning techniques cannot be free from such a trouble.

Making reference to countermeasures to the above, Fig. 4 shows setting a dose monitor 7 to dose of pre-incident X-ray according to a dilue by which the sensor output data should be corrected.

Specifically, monitor data are recorded in memory in the image processing zone with time-sequence without displaying the data in a n image picture and, after finishing photographing one image, corrective calculations will be added to sensor data. Or concurrently to photographing, corrective calculations will be added on real time basis to each sensing row, or appropriate measures will be added for controlling the scanning speed or count time so that incident dose be precisely defined for each scanning line. An important point is that inclusion of error with dose measurements will lead to variation in shade appearing in corresponding degree over one scanning line in a target image, by which viewing the target image is made difficult and a preventive thereto lies in considerable enhancement of sensibility with the dose monitor as compared with that of image sensors. If samesized sensors are used, outputs from several sensors should be added together to enhance and corrective calculations therefore should base on a standard value which is derived from four (4) data for example, each corresponding to the time measured at the dose monitor, because the output from one specific sensor is defined by addition of counts measured at different timings.

Further explanations of details of corrective calculations and transfer method of outputs to image memory, and the like are abbreviated from herein, because this art is similar to those generally used in usual image processings.

Descriptions up to here have been concerned with the case that measurements are conducted on digital circuits, though, analog devices would bring like advantages as noted above, within the scope of its nature. Underlying principles herein are equally applicable, not only to X-ray, as example of the radiation used, but also to gamma ray or visible light.

It is to be noted here that this invention may be modified or added readily with idea of extending spirits contained in the present invention, for instance, improvement in sensibility by increase of sensor rows and by diversifing functions with respective rows.

Conclusion

According to this invention, scanning time is largely reduced and worsening trend on an image is avoided in digital X-ray radiography. In particular, the embodiment noted as the second example hereinabove offers superior radiography wherein sensibility and resolution are improved to level of successful application, increase of sensors being held low, and disturbance due to scattered X-ray being held to a minimum.

Claims

I. Apparatus for radiographing an object body comprising a X-ray source (I), at least one slit (9) on one side of said object body (6) for controlling diffusion of X-ray emanated from the source, rows - (I4) of sensors (I8) on the other side of the object body (6) for detecting the transmitted X-ray, said apparatus further comprising drive means for moving the X-ray source (I), the slit (9) and the sensor rows (DI, ... Dn) relatively to said object body (6) while maintaining their relative position unchanged, so as to form an image on said rows of sensors by scanning said object body with said X-rays characterized in that said sensors (I8) are lined up in a plurality of rows (DI, ..., Dn) and are submitted to continuous exposure to X-ray during the scanning time corresponding to completing one image pattern, digital image data for the image of said object body being formed during said scanning time by accumulation of signals from the X-ray sensors (I8) defining image elements, said accumulation being built up by addition of signals from the X-ray sensors (I8) of different rows in agreement of sensing line therein with scanning spot.

2. Apparatus for radiography as defined in claim I, wherein the plurality of sensor rows are constituted of at least one pair of rows and wherein sensors belonging to one given pair of rows are disposed mutually shifted in such a way that the scanning lines for one row and the scanring lines for the other are interlaced.

3. Apparatus for radiography as defined in claim 2, wherein a plurality of slits are provided, each slit cooperating with at least one respective row of sensors.

4. Apparatus for radiography as defined in claim 2 or 3, wherein the sensors of adjacent rows are disposed so that void portions in one row do not appear at the same position as the void portions in the other row.

5. Apparatus for radiography as defined in claim I or 2, wherein the X-ray source is a X-ray tube and a dose monitor is provided to monitor timewise fluctuation in intensity of X-ray emanated from the X-ray tube.

6. Apparatus for radiography as defined in claim I or 2 or 3, wherein the sensor rows are disposed adjacent to each other with an interval greater than one image element length.

**FIG 1**

accumulation  read-out

$D1(k):$ (W1) (W2) (W3) (W4)

$D2(k):$ (W1) (W2) (W3)

$D3(k):$ (W1) (W2)

$\vdots$ : (W1)

$Dn(k):$ (W1)

$0 \quad \dfrac{d}{v} \quad \dfrac{2d}{v} \quad \dfrac{3d}{v} \quad \dfrac{4d}{v} \quad \dfrac{nd}{v} \quad t$

$$\dfrac{(n-1)d}{v}$$

**FIG 2**

0 230 155

FIG 3

**FIG** 4

FIG 6

before scanning

scanning

after scanning

**FIG** 7

## DOCUMENTS CONSIDERED TO BE RELEVANT

| Category | Citation of document with indication, where appropriate, of relevant passages | Relevant to claim | · CLASSIFICATION OF THE APPLICATION (Int. Cl.4) |
|---|---|---|---|
| Y | US-A-4 203 037 (GUR et al.) <br> * Figures 3,5,7; column 5, line 6 - column 6, line 11; column 6, line 46 - column 7, line 32; column 9, line 16 - column 10, line 8 * | 1 | A 61 B 6/06 <br> A 61 B 6/03 <br> G 01 T 1/164 |
| A | | 3 | |
| | --- | | |
| Y | US-A-4 383 327 (KRUGER) <br> * Figures 1,3,4; column 6, line 7 - column 8, line 38; column 11, line 14 - column 12, line 35 * | 1 | |
| | --- | | |
| A | US-A-4 179 100 (SASHIN et al.) <br> * Figures 4,5; column 8, line 54 - column 9, line 80; column 10, line 53 - column 11, line 12 * | 3 | TECHNICAL FIELDS SEARCHED (Int. Cl.4) |
| | --- | | A 61 B |
| A | EP-A-0 057 957 (PHILIPS PATENTVERWALTUNG GmbH) <br> * Figures; page 5, line 29 - page 6, line 18 * | 1,3,5 | G 21 K <br> G 01 T |
| | --- | | |
| A | GB-A-1 406 685 (BAIRD-ATOMIC INC.) <br> * Figure 1; page 2, lines 77-97 * | 2 | |
| | --- -/- | | |

The present search report has been drawn up for all claims

| Place of search | Date of completion of the search | Examiner |
|---|---|---|
| THE HAGUE | 20-03-1987 | CHEN A.H. |

| | **DOCUMENTS CONSIDERED TO BE RELEVANT** | | Page 2 |
|---|---|---|---|
| Category | Citation of document with indication, where appropriate, of relevant passages | Relevant to claim | CLASSIFICATION OF THE APPLICATION (Int. Cl.4) |
| A | IEEE TRANSACTIONS ON NUCLEAR SCIENCE, vol. NS-27, no. 4, August 1980, pages 1305-1311, IEEE, New York, US; J.A. McINTYRE: "Design features of a positron tomograph with 2.4 mm resolution" <br> * page 1305, abstract; page 1306, figure 2, section "General description" * <br><br> ----- | 6 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 20-03-1987 | CHEN A.H. |

EPO Form 1503. 03.82